Europäisches Patentamt

European Patent Office

Office européen des brevets

(11) Publication number: **0 244 456 B1**

## EUROPEAN PATENT SPECIFICATION

(45) Date of publication of patent specification: **15.01.92** (51) Int. Cl.5: **A61M 5/315**, A61K 9/52, B01J 13/02

(21) Application number: **86906578.9**

(22) Date of filing: **08.10.86**

(86) International application number:
**PCT/US86/02128**

(87) International publication number:
**WO 87/02253 (23.04.87 87/09)**

(54) **IN SITU PREPARATION AND DELIVERY OF LIPOSOMES.**

(30) Priority: **21.10.85 US 789410**

(43) Date of publication of application:
**11.11.87 Bulletin 87/46**

(45) Publication of the grant of the patent:
**15.01.92 Bulletin 92/03**

(84) Designated Contracting States:
**AT BE CH DE FR GB IT LI LU NL SE**

(56) References cited:
**EP-A- 0 152 379**
**US-A- 4 599 227**

(73) Proprietor: **THE LIPOSOME COMPANY, INC.**
**One Research Way Princeton Forrestal Center**
**Princeton, NJ 08540(US)**

(72) Inventor: **POPESCU, Mircea, C.**
**5 Parkway Avenue**
**Plainsboro, NJ 08536(US)**

(74) Representative: **Ahner, Francis et al**
**CABINET REGIMBEAU, 26, avenue Kléber**
**F-75116 Paris(FR)**

Rank Xerox (UK) Business Services

**Description**

BACKGROUND OF THE INVENTION

The present invention relates to the preparation, storage, and hydration of a lipid film in a syringe. More particularly, it is concerned with coating lipids on the inner surface of the syringe and hydrating the lipid films immediately prior to use.

Liposomes are completely closed structures composed of lipid bilayer membranes containing an encapsulated aqueous volume. Liposomes may contain many concentric lipid bilayers separated by aqueous phase (multilamellar vesicles or MLVs), or may be composed of a single membrane bilayer (unilamellar vesicles).

Liposome preparation has typically been achieved by the process of Bangham et.al., (1965 J. Mol. Biol., 13: 238-252) whereby lipids suspended in organic solvent are evaporated under reduced pressure to a dry film in a reaction vessel. An appropriate amount of aqueous phase is then added to the vessel and the mixture agitated, then allowed to sit undisturbed for a period of time, forming MLVs. The aqueous phase entrapped within the liposomes may comprise bioactive agents including drugs, hormones, proteins, dyes, vitamins, or imaging agents.

The liposome preparation method of the present invention allows one receptacle to serve as the reaction, storage, and delivery vessel. The procedure therefore reduces the steps necessary for formation and delivery of liposomes when compared to standard formation procedures which employ large volume flasks and comprise the steps of: (a) drying the lipid under vacuum rotoevaporation with later hydration of the film in the flask; (b) storing the formed liposomes in suitably small receptacles; and (c) transferring the liposomes to a syringe for use. This invention enables decreased manipulation and transfer of the preparation while simultaneously addressing the problem of lipid stability, as lipid films rather than hydrated liposomes are the stored entity. US 4,308,166 discloses the use of a syringe as an emulsification device. EP-A-152379 discloses the preparation of a lyophilized lipisomal Formulation in a vial.

SUMMARY OF THE INVENTION

The present invention describes a method for in situ preparation of liposomes in a syringe comprising the steps of: (a) removing solvent from a solvent-lipid mixture in the syringe to obtain a lipid film on the inner surface of the syringe; (b) sealing the syringe so that the lipid film is in an inert atmosphere; and (c) hydrating the lipid film to form liposomes which are ready for immediate use. Both lipophilic and hydrophilic substances may be encapsulated in the liposomes by the steps of (1) drying the lipophilic drug or bioactive agent onto receptacle sides with the lipid film; or (2) employing aqueous buffer that additionally contains a hydrophilic drug or bioactive agent. Lipids are dried to a film in a small receptacle such as a tube or syringe, then stored and hydrated in the same receptacle. This procedure reduces handling, as well as storage volume necessary to maintain liposome preparations.

DETAILED DESCRIPTION OF THE INVENTION

The present invention describes a preparation and storage procedure for amphipathic compounds which are hydrated to form liposomes. Amphipathic compounds are those which contain both hydrophobic and hydrophilic groups. Amphiphathic compounds which may be employed in the present invention include glycolipids, phospholipids, sphingomyelin, and sterols such as cholesterol which may be combined with the phospholipids. These lipids are deposited on the sides of a syringes by removal of the suspending solvent. Should the lipid be suspended in a toxic solvent such as chloroform, diethyl ether, methanol or methylene chloride, the solvent may be removed under reduced pressure and the lipid resuspended in a pharmaceutically accepted solvent (i.e., one that is non-toxic) such as ethanol. In the preferred embodiment, the lipid is dried from its suspending solvent and re-solubilized to about 100 mg/ml in absolute ethanol. Lipids suspended in such a solvent are placed into the syringe and dried to a thin film by use of a rotoevaporator under reduced pressure, fully coating the receptacle sides with the lipid suspension. If the material to be entrapped is lipophilic, it can be dried down onto the receptacle inner surface with the lipid.

The lipids and amounts thereof employed depend on the nature of the bioactive agent(s) added to the preparation, and the preferred characteristics of the resulting liposomes. The bioactive agent to be encapsulated may be soluble in organic solvent, and therefore suspended in the organic solvent-lipid mixture prior to evaporation. Alternatively, the bioactive agent may be soluble in aqueous phase, and is therefore introduced to the dried lipid film upon hydration. Thirdly, a bioactive agent partially soluble in organic solvent may be suspended in aqueous solvent and then mixed with the organic solvent prior to evaporation.

Following the formation of a uniform lipid film on the inner surface of the syringe, the syringe sealed in an inert environment (one substantially free of oxygen and water to prevent oxidation and partial hydration). Preferred inert environments include nitrogen, argon, or vacuum. The syringe plunger is preferably inserted into the barrel and fully compressed, thus depositing the lipids at the delivery end of the syringe, and effectively sealing the head end of the syringe. The delivery end of the syringe may be sealed by any suitable method known in the art.

Hydrating the lipid film is accomplished by exposing the film to an aqueous medium such as distilled water or aqueous buffer; for example, phosphate buffered saline (PBS), tris(hydroxymethyl)aminomethane (Tris), or (N-2-hydroxyethyl piperazine-N'-2-ethane sulfonic acid) (HEPES) at pH between about 7.0-7.5, more preferably at pH about 7.2. If the material to be entrapped is hydrophilic it can be dissolved in the aqueous medium. An aliquot of aqueous medium is drawn into the syringe and mixed with the lipid film. Generally, 2 to 3 plunges of the syringe piston, brief agitation, and undisturbed standing for several minutes will result in liposome formation. The resulting liposomes can be administered parenterally to a subject including mammals such as humans.

EXAMPLE 1

Twenty milligrams of egg phosphatidylcholine (EPC) (Sigma Chemical Co., St. Louis, MO) was dissolved in absolute ethanol to form a solution at a concentration of 100 mg/ml. This solution was combined with 0.8 ml of 100% ethanol, and 20 $\mu$l of a 7% aqueous solution of Arsenazo III (Sigma Chemical Co., St. Louis, MO) in both 13 X 100 mm borosilicate glass and polystyrene tubes. The tubes were attached to a rotoevaporator by an adapter system consisting of two stoppers of appropriate size through which holes have been bored to accommodate a connecting tube. The stoppers are inserted into the appropriate vessel; receptacle and rotoevaporator. The mixtures were dried in a horizontal position by vacuum rotoevaporation at 0.1 millitorr, at 37°C, and at 22 rpm followed by 154 rpm until a uniformly thin lipid/Arsenazo III film was deposited on the sides of the tube.

EXAMPLE 2

The procedures and materials of Example 1 were employed omitting the Arsenazo III from the preparation. A white lipid film was uniformly deposited on the walls of both the glass and plastic tubes.

EXAMPLE 3

The procedures in Example 1 were employed with the materials listed in Table 1. Both 10cc and 20cc polypropylene syringes were employed.

Table 1

| EPC | Ethanol | PBS(pH 7.2) | Arsenazo III |
|---|---|---|---|
| 50 mg | 1.0 ml | - | 20$\mu$l |
| 50 mg | 1.0 ml | 50$\mu$l | 20$\mu$l |
| 100 mg | 0.5 ml | - | 20$\mu$l |
| 100 mg | 0.5 ml | 50$\mu$l | 20$\mu$l |

Following rotoevaporation of the lipid to a film, the plunger was inserted into the syringe barrel and the lipid removed to the bottom of the syringe.

Liposomes were formed by drawing PBS buffer at pH 7.2 into the syringe to a volume of 5% of the total syringe capacity, agitating the syringe, and allowing several minutes for liposome formation.

Entrapment was visually observed by centrifuging the syringe-prepared liposomes to a pellet, and noting Arsenazo III color intensity. Color intensity was compared to that of centrifuged MLVs and SPLVs prepared by standard technique. Liposomes prepared by syringe technique demonstrated equal or greater color intensity and therefore entrapment.

EXAMPLE 4

3

The procedures and materials of Example 1 are employed with the additional step of sealing the tube. A rubber septum is inserted into the mouth of the tube. Two needles are inserted through the diaphragm of the septum, serving as inlet and outlet ports. The needle serving as the inlet port is attached to a pressurized nitrogen cylinder by way of a connecting hose. The nitrogen valve is turned on allowing a steady stream of nitrogen to flow through the sample tube for 5 minutes, purging the air. After this time, the outlet needle is removed, followed by the inlet needle, leaving a slight positive nitrogen pressure in the tube.

**Claims**

1. A method for in situ preparation of liposomes in syringe comprising the steps of:
   a) removing solvent from a solvent-lipid mixture in the syringe to obtain a film on the inner surface of the syringe;
   b) sealing the syringe so that the film is in an inert atmosphere; and
   c) hydrating the film to form liposomes.

2. The method of claim 1 wherein the lipid comprises glycolipids, phospholipids, sphingomyelin or sterols.

3. The method according to either of claims 1 or 2 comprising the additional step of fully compressing the syringe plunger, thereby depositing the lipid at the syringe base.

4. The method according to any preceding claim comprising the additional step of hydrating the lipid film by drawing aqueous buffer into the syringe.

5. The method according to any preceding claim wherein the hydrating step employs aqueous buffer.

6. The method according to any preceding claim wherein the aqueous buffer additionally comprises a bioactive agent.

7. A syringe for delivering a lipid composition comprising:
   a) a barrel, the inner surface of which is coated with a film comprising a lipid ;
   b) a movable plunger inserted in the barrel thus providing a seal for the contents at the head end of the syringe; and
   c) a seal at the delivery end of the syringe.

8. The syringe according to claim 7 wherein the lipid comprises glycolipids, phospholipids, sphingomyelin or sterols.

9. The syringe according to claim 8 where in the lipid comprises phospholipid.

10. The syringe of anyone of claims 7 to 9 wherein the film additionally comprises a bioactive agent.

**Revendications**

1. Procédé de préparation in situ de liposomes dans une seringue, comprenant les étapes de :
   a) enlèvement du solvant d'un mélange solvant-lipide dans la seringue pour obtenir une pellicule à la surface interne de la seringue ;
   b) scellement de la seringue, de manière que la pellicule soit dans une atmosphère inerte ; et
   c) hydratation de la pellicule pour former des liposomes.

2. Procédé de la revendication 1, dans lequel le lipide comprend des glycolipides, des phospholipides, de la sphingomyéline ou des stérols.

3. Procédé selon l'une quelconque des revendications 1 ou 2, comprenant l'étape additionnelle de pleine compression du piston de la seringue, déposant ainsi les lipides à la base de la seringue.

4. Procédé selon l'une quelconque des revendications précédentes, comprenant l'étape supplémentaire d'hydratation de la pellicule de lipide par aspiration d'un tampon aqueux dans la seringue.

4

5. Procédé selon l'une quelconque des revendications précédentes dans lequel l'étape d'hydratation emploie un tampon aqueux.

6. Procédé selon l'une quelconque des revendications précédentes, dans lequel le tampon aqueux comprend en outre un agent bioactif.

7. Seringue pour fournir une composition lipidique comprenant :
   a) un cylindre, dont la surface interne est revêtue d'une pellicule comprenant un lipide ;
   b) un piston mobile inséré dans le cylindre, fournissant ainsi un scellement pour le contenu à l'extrémité de tête de la seringue ; et
   c) un scellement ou joint à l'extrémité de fourniture de la seringue.

8. Seringue selon la revendication 7, dans laquelle le lipide comprend des glycolipides, phospholipides, de la sphingomyéline ou des stérols.

9. Seringue selon la revendication 8, dans laquelle le lipide comprend un phospholipide.

10. Seringue de l'une quelconque des revendications 7 à 9, dans laquelle la pellicule comprend en outre un agent bioactif.

**Patentansprüche**

1. Verfahren zur Herstellung von Liposomen in situ in einer Injektionsspritze, umfassend die Stufen:
   a) Entfernen von Lösungsmittel aus einem Lösungsmittel-Lipid-Gemisch in der Injektionsspritze um einen Film an der inneren Oberfläche der Injektionsspritze zu erhalten;
   b) Verschließen der Injektionsspritze, so daß der Film in einer innerten Atmosphäre vorliegt; und
   c) Hydratisieren des Films zur Bildung von Liposomen.

2. Verfahren nach Anspruch 1, bei dem das Lipid Glycolipide, Phospholipide` Sphingomyelin oder Sterole umfaßt.

3. Verfahren nach einem der Ansprüche 1 oder 2, umfassend die zusätzliche Stufe der vollen Kompression des Kolbens der Injektionsspritze, wodurch das Lipid an der Basis der Injektionsspritze abgelagert wird.

4. Verfahren nach einem der vorhergehenden Ansprüche, umfassend die zusätzliche Stufe des Hydratisierens des Lipidfilms durch Einziehen von wäßrigem Puffer in die Injektionsspritze.

5. Verfahren nach einem der vorhergehenden Ansprüche, bei dem die Hydratisierstufe unter Verwendung von wäßrigem Puffer durchgeführt wird.

6. Verfahren nach einem der vorhergehenden Ansprüche, bei dem der wäßrige Puffer zusätzlich ein bioaktives Mittel umfaßt.

7. Injektionsspritze zur Abgabe einer Lipid Zusammensetzung, umfassend:
   a) einen Zylinder, dessen innere Oberfläche mit einem Film ausgekleidet ist, der ein Lipid umfaßt;
   b) einen in den Zylinder eingeführten beweglichen Kolben, um den Inhalt am Kopfende der Injektionsspritze abzuschließen;
   c) einen Verschluß am Abgabeende der Injektionsspritze.

8. Injektionsspritze nach Anspruch 7, worin das Lipid Glycolipide, Phospholipide, Sphingomyelin oder Sterole umfaßt.

9. Injektionsspritze nach Anspruch 8, worin das Lipid Phospholipid umfaßt.

10. Injektionsspritze nach einem der Ansprüche 7 bis 9, worin der Film zusätzlich ein bioaktives Mittel umfaßt.

5